# EUROPEAN PATENT APPLICATION

(11) **EP 0 776 666 A2**
(43) Date of publication of application: **04.06.1997**
(21) Application number: 96106837.6
(22) Date of filing: 30.04.1996
(51) Int. Cl.: A61K 35/78, A61K 31/12, A61K 31/045

(54) **Analgesic anti-inflammatory drug**

(30) Priority: 30.11.1995 JP 336105/95
(71) Applicant: NIPPON MEKTRON, LTD., Minato-ku Tokyo (JP)
(72) Inventor: Nakamura, Motoyuki, Kitaibaraki city, Ibaraki (JP); Nakasumi, Tetsuo, Mirandopolis Capit. Sao Paolo 04049-050 (BR); Yoshizawa, Toyokichi, Kitaibaraki city, Ibaraki (JP); Minagawa, Yumiko, Kitaibaraki city, Ibaraki (JP)
(74) Representative: Hansen, Bernd, Dr. Dipl.-Chem.

(57) **Abstract**

An analgesic-anti-inflammatory drug comprising quercetin-3-0-glucoside or a friedelan type compound (friedelin, friedelan-3-α-ol or friedelan-3-β-ol) separated from the alcohol extract of Maytenus ilicifolia as an effective component. The friedelan type compound has been already found to have an anti-ulcerative effect and therefore can be used as an analgesic-anti-inflammatory drug without any side effect such as gastrointestinal disorder (generation of ulcer), etc. as caused by a non-steroid anti-inflammatory analgesics.

## Description

### BACKGROUND OF THE INVENTION

### 1. FIELD OF THE INVENTION

The present invention relates to an analgesic-anti-inflammatory drug, and more particularly to an analgesic-anti-inflammatory drug comprising a plant extract as an effective component.

### 2. RELATED PRIOR ART

Medical treatment of severely painful diseases, particularly end stage cancer causing a cancer pain is an important medical care. The cancer pain is caused by tumors perse, by side effects due to the cancer treatment or by psychogenic pain and is often treated according to the Brompton's cocktail prescription and the WHO prescription.

In both prescriptions, administration starts with a small dose and is continued with an increasing dose thereof until the pain can be alleviated fully and morphine is used as a basic drug ingredient. Thus, the continuous administration causes to form a tolerance, and consequently form considerable mental and physical drug dependence. Once such chronic addiction symtoms are identified, sudden abstinence symptoms are exhibited by cessation of administration of the drug, resulting in recurrence of pains and also deterioration of mental and physical functions, such as anxiety, phobias, insomnia, etc.

Up to now, so many compounds showing an analgesic-anti-inflammatory action against the cancer pain have been reported, and poppy (Papaver somuniferum) is particularly well known as a crude drugs showing the analgesic-anti-inflammatory action. Poppy (Papaver somuniferum) has highly effective analgesic, antispasmodic, anticough and antidiarrheic actions, but is designated narcotics due to a high level habituation. The main components of opium obtained from the poppy are morphine-based opium alkaloids and morphine derivatives, and their continuous administration inevitably gives rise to various side effects due to their nature, as described above.

### SUMMARY OF THE INVENTION

An object of the present invention is to provide an analgesic-anti-inflammatory drug comprising an extract from plants having no such side effects as an effective component.

According to the present invention, there is provided an analgesic-anti-inflammatory drug comprising an aqueous layer component obtained by intersolvent distribution of alcohol extract of Maytenus ilicifolia between chloroform and water as an effective component.

### DETAILED DESCRIPTION OF THE INVENTION

Maytenus ilicifolia is a plant of the family Celastraceae used as a folk medicine in Brazil and known as a specific remedy for gastric ulcer, duodenal ulcer, intestinal fermentation, etc. In the present invention, its leaves are used, but it has not been known at all that its extract has a remarkable analgesic-anti-inflammatory effect.

Alcohol extraction is carried out as follows:
Raw leaves, or dried and pulverized leaves of Maytenus ilicifolia are extracted with an alcohol such as methanol, ethanol, n-butanol, etc., preferably methanol, usually under reflux condition and then alcohol is distilled off from the extract to obtain a dark green powdery alcohol extract.

The thus obtained alcohol extract is subjected to intersolvent distribution between chloroform and water (purified water) and the resulting aqueous layer component is concentrated under reduced pressure, whereby yellowish brown powders can be obtained. The yellowish brown powders are apllied to various chromatographies and successive elutions with water, alcohol or ketone of various concentrations, whereby fractions having a high analgesic-anti-inflammatory effect can be obtained. More specificelly, the yellowish brown powders obtained from the aqueous layer component are dissolved into water and the resulting aqueous solution is apllied to a column chromatography using polystyrene-based resin and elution with water and alcohol or ketone of various concentrations, whereby particularly a 100% methanol eluate fraction and a 50% acetone eluate fraction having a distinguished analgesic-anti-inflammatory effect can be obtained.

When the 100% methanol eluate fraction was further apllied to chromatography using Sephadex (polysaccharide dextran-based beads) and to fractionation using methanol as an eluent, a fraction identified as quercetin-3-0-glucoside (isoquercitrin) having a particularly distinguished analgesic-anti-inflammatory can be obtained.

Furthermore, when the solvent, i. e. chloroform is distilled off from the chloroform layer component obtained by the intersolvent distribution of the alcohol extract between the water and the chloroform, dark green oily substance can be obtained. When the thus obtained oily substance from the chloroform layer component are dissolved in n-hexane, and the solvent, i. e. n-hexane, is distilled off from the resulting solution, dark green oily substance can be obtained, whereas black oily substance can be obtained from the insoluble residue.

When the n-hexane-insoluble black oily substance are applied to silicagel chromatography and elute with chloroform to conduct fractionation into fractions Nos. 1 to 100, each in 20ml, friedelan-3-on (friedelin) can be obtained from fractions Nos. 10 to 15, and friedelan-3-β-ol (epifriedelinol) can be obtained from fractions Nos. 24 to 50. These compounds are known compounds and identification of friedelan-3-β-ol having an anti-ulcerative effect has been conducted by comparison of its physico-chemical properties. When friedelan-3-on is reduced by metallic sodium or the like as a reducing agent according to a known reduction procedure, friedelan-3-α-ol (friedelinol) also as a known compound ca be obtained.

It was found that these three friedelan type compounds had an analgesic-anti-inflammatory effect on pains and inflammations induced by formalin injection to soles of mouse rear paws. Furthermore, in case of friedelan-3-β-ol, its action is antagonized by premedication of naloxone as a morphine antagonist, and thus this suggests that friedelan-3-β-ol acts on an opioid receptor like morphine to exhibit an analgesic-anti-inflammatory effect.

These extracts can be provided in the form of powder, granules, tablets, sugar coated tablets, capsules, liquid preparations or the like and can be administered perorally, parenterally, by inhalation, by perrectum, locally or the like. The parenteral administration includes a subcutaneous injection, an intravenous injection, an intramuscular injection, an intranasal administration or injection. A dose is usually in a range of about 0.1 to about 500 mg/kg body weight, and an exact dose is determined within the above-mentioned range, depending on ages, body weights and symptoms of patients and also administration route, etc. and administration is made usually one to five times a day. Their toxicity is low and investigation of acute toxicity on male Wistar rats by oral administration revealed that no killing occurred even at 3,000 mg/kg (p. o.)

The present invention provides an analgesic-anti-inflammatory drug comprising quercetin-3-0-glucoside or a friedelan type compound extracted from Maytenus ilicifolia as an effective component. The plant Maytenus ilicifolia has been so far used as a folk medicine and therefore has no fear of chronic addiction symptom, etc. Furthermore, the analgesic-anti-inflammatory action of the present drug is quite similar to that exhibited so far by narcotic analgesics such as morphine, etc. (first phase reaction), and thus naturally has such an analgesic-anti-inflammatory action as exhibited by ordinary analgesics such as aspirin, etc. (second phase reaction). That is, the present drug can exhibit a distinguished analgesic-anti-inflammatory action not only on headache, abdominal pain, neuralgia, etc., but also on cancer pain. Furthermore, it was found that the friedelan type compounds had also an anti-ulcerative effect. Therefore, the present drug can be used as an analgesic-anti-inflammatory drug without any side effect such as gastrointestinal disorder (generation of ulcer), caused by non-steroid anti-inflammatory analgesics.

### BRIEF DESCRIPTION OF THE DRAWINGS

Fig. 1 is a graph showing results of acetic acid writhing tests of Samples 1 to 7.

Fig. 2 is a graph showing results of formalin tests of Samples 1 to 7.

Fig. 3 is a graph showing results of acetic acid writhing test of Sample 8.

Fig. 4 is a graph showing results of formalin test of Sample 8.

Fig. 5 is a graph showing results of formalin test (a).

Fig. 6 is a graph showing results of formalin test (b).

Fig. 7 is a graph showing results of formalin test (c).

Fig. 8 is a graph showing results of naroxone premedication-formalin test (d).

Fig. 9 is a graph showing results of formalin test (e).

Fig. 10 is a graph showing results of formalin test (f).

Fig. 11 is a graph showing results of naroxone premedication-formalin test (g).

Fig. 12 is a graph showing results of formalin test (h).

### PREFERRED EMBODIMENTS OF THE INVENTION

The present invention will be described in detail below, referring to Example.

### EXAMPLE

3.2kg of dried crude Maytenus ilicifolia was twice extracted with 30 liters of methanol with heating at the reflux temperature, and then the solvent was distilled off, whereby 153.3g of methanol extract (Sample 1) was obtained. The methanol extract was subjected to intersolvent distribution between chloroform and water, and the respective solvents were distilled off, whereby 58.0g of dark green oily substance (Sample 2) was obtained from the chloroform layer component and 82.9g of yellowish brown powders (Sample 3) was obtained from the aqueous layer component.
(1) Sample 3 obtained from the aqueous layer component was dissolved in 300ml of water and the resulting solution was applied to chromatography using 1,000ml of polystyrene-based resin (styrene-divinylbenzene-based copolymer resin, Diaion HP-20, trademark of a product made by Mitsubishi Kasei K. K., Japan) and to fractionation into the following 4 eluate fractions, each in 1,000ml.

| | |
|---|---|
| Sample 4: H₂O eluate fraction | 58.8g |
| Sample 5: 50% MeOH eluate fraction | 8.7g |
| Sample 6: 100% MeOH eluate fraction | 4.5g |
| Sample 7: 50% Me₂CO eluate fraction | 10mg |

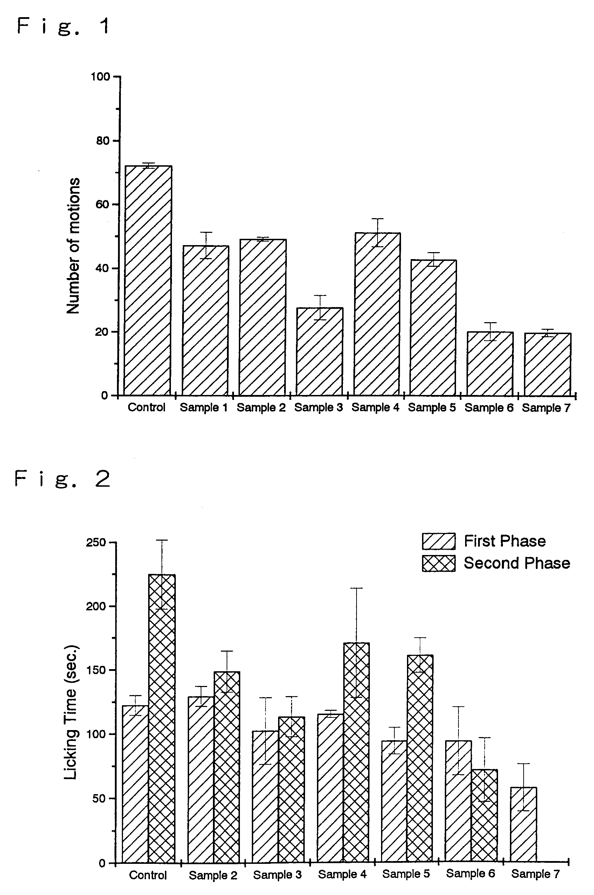

The foregoing extraction and fractionation samples were subjected to the following analgesic-anti-inflammatory action tests (acetic acid writhing tests and formalin tests), and it was found that Samples 3, 6 and 7 had a high activity in the acetic acid writhing tests and Samples 6 and 7 had a particularly high activity in the formalin tests.
Acetic acid writhing test:
When 500µl of 1% acetic acid as a stimulant was injected into a mouse abdominal cavity, specific agony expressions such as twisting motions (writhing) of lower part of the body, or motions to flatten the stomach and stretch the rear paws were observable. On the basis of these phenomena, number of occurrences of such motions was counted over 15 minutes from the time of 5 minutes after the injection of 1% acetic acid and compared with that of the control administered with physiological saline. 1mg of one of the Samples was dissolved into 200µl of 10% dimethylsulfoxide, and the thus prepared solution was administered subcutaneously at a dose of 1mg/mouse at the time of 10 minutes before the injection of the stimulant. The results (one group consiting of 5 mice) are shown in the following Table 1 and Fig. 1.

**TABLE 1**

| | Number of occurrence of motions |
|---|---|
| Control | 72.3±0.85 |
| Sample 1 | 47.3±4.09 |
| Sample 2 | 49.3±0.63 |
| Sample 3 | 27.8±3.86 |
| Sample 4 | 51.3±4.38 |
| Sample 5 | 43.0±2.16 |
| Sample 6 | 20.3±2.85 |
| Sample 7 | 20.0±1.16 |

Formalin test (analgesic-anti-inflammatory effect test):
100µl of 2% formalin was injected as a stimulant into the soles of rear paws (subcutaneous tissues of paw soles) of a mouse, and total licking time of the mouse, i.e. duration of motions for a mouse to lick the rear paws to relieve the pain, was measured by a stopwatch for each 5 minutes and continuously over 30 minutes. In the measurement, 0.2mg of each of Samples were dissolved into 200µl of 10% dimethylsulfoxide, respectively, and the thus prepared each solutions were administered into the abdominal cavities, respectively, at a dose of 0.2mg/mouse at the time of 10 minutes before the injection of the stimulant.
In the formalin test, two reactions, i. e. first phase reaction and second phase reaction, occur. The first phase reaction is occurrence of pains in 0 to 10 minutes after the formalin stimulation, i. e. that of pains caused by direct stimulation of sensory nerve peripheries by the formalin injection. Drugs acting to control such pains sofar used have been only narcotic analgesics such as morphine, etc., whose action seems to be on the central nerve.
The second phase reaction is occurrence of pains in 10 to 30 minutes after the formalin injection, i. e. that of pains arise from to inflammation caused by the formalin injection. The second phase reaction can be controlled by the ordinary analgesics such as aspirin, etc.
Thus the evaluation was carried out in two phases, i. e. a first phase from 0 to 10 minutes and a second phase from 10 to 30 minutes, and total licking time in seconds, that is, duration of motions for a mouse to lick the rear paws, was measured and compared with control.
Results of the formalin test for groups each of 5 mice are shown in the following Table 2 and Fig. 2.

**TABLE 2**

| | First phase of 0 - 10 mins. | Second phase of 10 - 30 mins. |
|---|---|---|
| Control | 122.61±7.74 secs. | 224.82±27.02 secs. |
| Sample 2 | 129.50±7.92 secs. | 148.87±16.19 secs. |
| Sample 3 | 102.32±26.1 secs. | 113.48±15.77 secs. |
| Sample 4 | 115.31±2.70 secs. | 170.67±42.75 secs. |
| Sample 5 | 94.17±10.59 secs. | 161.10±13.51 secs. |
| Sample 6 | 94.08±26.55 secs. | 71.53±24.72 secs. |
| Sample 7 | 57.91±18.18 secs. | 0.00±0.00 sec. |

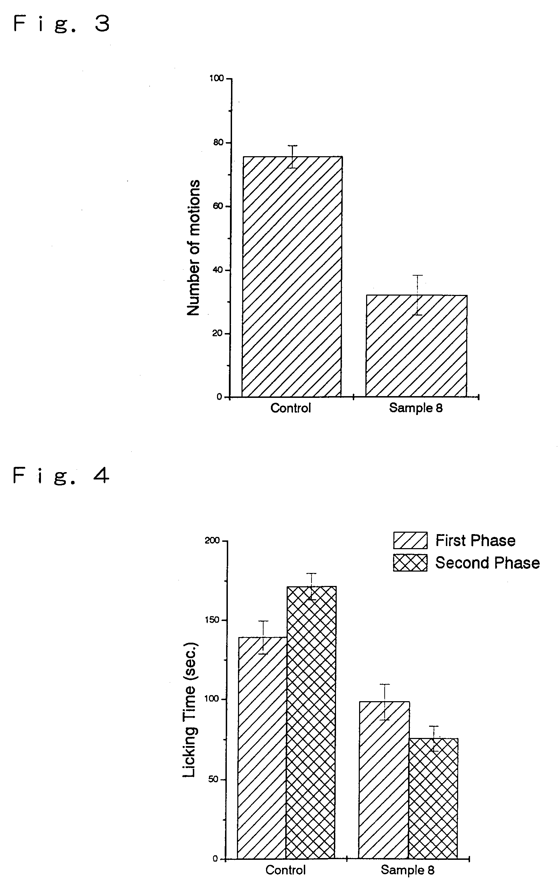

(2) From Samples 6 and 7 having a high activity, as shown in Table 2, Sample 6 was selected and applied to column chromatography using 100g of Sephadex LH-20 (trademark of a product made by Pharmacia) and fractionated into fractions Nos. 0 to 120, each in 100ml, using methanol as an eluent.
The thus obtained fractions were each subjected to analgesic-anti-inflammatory effect tests and it was found that 15.4mg of yellow powder (Sample 8) obtained from fractions Nos. 43 to 55 was effective, as given below and in Figs. 3 and 4:
(Acetic acid writhing test ; Number of occurrence of motions)
- Control: 75.6±3.54
- Sample 8: 32.1±6.27
(Formalin test)
First phase of 0 to 10 minutes
- Control: 139.09±10.52 secs.
- Sample 8: 98.14±11.25 secs.
Second phase of 10 to 30 minutes
- Control: 171.18±8.35 secs.
- Sample 8: 75.36±7.78 secs.

Sample 8 had the following physico-chemical properties:
- ¹H-NMR(DMSO-d₆)δ :: 7.71(1H, brs), 7.58(1H,d,J=8.58), 6.87(1H, d, J=8.58), 6.38(1H,brs), 6.19(1H,brs), 5.23(1H, d,J=7.26)
- ¹³C-NMR(DMSO-d₆)δ:: 179.6, 166.1, 163.1, 158.5, 149.9, 146.0, 135.7, 123.3, 123.2, 117.7, 117.6, 116.1, 104.5, 102.4, 100.0, 94.8, 78.4, 78.2, 75.8, 71.3, 62.6

These physico-chemical properties were found to be indentical with those disclosed in 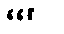 The Flavonoids: advances in research", published by Chapman and Hall in 1982, and thus identified to be quercetin-3-0-glucoside (isoquercitrin).
(3) Said Sample 2 was dissolved in n-hexane, and the solvent was distilled off from the solution, whereby 12.0g of dark green oily substance (Sample 9) was obtained, whereas 41. 9g of black oily substance (Sample 10) was obtained from the undissolved part.
11g of this Sample 10 was subjected to chromatography using 100ml of silica gel column (Kieselgel Art 7734, trademark of a product made by Merck Co.) and elute with chloroform to fractionate the eluate into fractions Nos. 1 to 100, each in 20ml.
116mg of light yellow crystal was obtained from fractions Nos. 10 to 15 and recrystallized from chloroform, whereby 90mg of colorless needle-like crystal (Sample 11) was obtained.
- ¹³C-NMR(CDCl₃-d)δ:: 213.2(s), 59.5(d), 58.2(d), 53.1(d), 42.8(d), 42.1(s), 41.5(t), 41.3(t), 39.7(s), 39.2(t), 38.3(s), 37.4(s), 36.0(t), 35.6(t), 35.3(t), 35.0(q), 32.7(t), 32.4(t), 32.1(q), 31.8(q), 30.5(t), 30.0(s), 28.2(s), 22.3(t), 20.2(q), 18.7(q), 18.2(t), 17.9(q), 14.6(q), 6.8(q)

The foregoing physico-chemical properties were found to be identical with those of friedelan-3-on (friedelin) represented by the following chemical formula, as disclosed in Magn. Reson. Chem. 23, page 616 (1985) and 25, page 39 (1987). 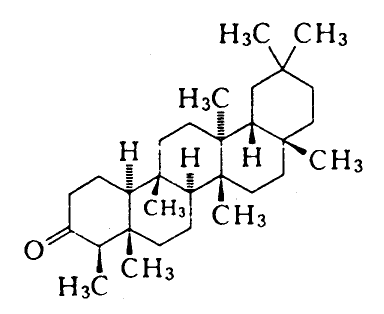
Furthermore, 550mg of light yellowish green powder was obtained from fractions Nos. 24 to 50, and recrystallized from chloroform, whereby 470mg of colorless crystal (Sample 12) was obtained.
[α]_{D}21.4°(c=0.33, CHCl₃)
HR-MS: m/z 428.4024[M]
- ¹³C-NMR(CDCl₃-d)δ:: 72.8(d), 61.4(d), 53.2(d), 49.2(d), 42.9(d), 41.8(t), 39.7(s), 39.3(t), 38.4(s), 37.9(s), 37.1(s), 36.1(t), 35.6(t), 35.4(t), 35.2(t), 35.0(q), 32.8(t), 32.4(t), 32.1(q), 31.8(q), 30.7(t), 30.0(s), 28.2(s), 20.1(q), 18.7(q), 18.3(q), 17.6(t), 16.4(q), 15.8(t), 11.6(q)

The foregoing physico-chemical properties were found to be identical with those of friedelan-3-β-ol (epifriedelinol) represented by the following chemical formula, as disclosed in QUIM ICA NOVA, 31, No.4, page 254 (1990) and J. Am. Chem. Soc., 78, page 5041 (1956). 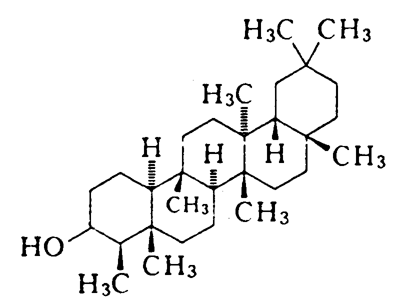
When 100mg of Sample 11 was subjected to reduction with metallic sodium as a reducing agent in reflux amyl alcohol according to the method disclosed above J. Am. Chem. Soc., 70mg of colorless powder (Sample 13) having the following physico-chemical properties was obtained and identified to be friedelan-3-α-ol (friedelinol) having the following chemical formula:
[α]_{D}10.9°(c=0.18, CHCl₃)

- ¹³C-NMR(pyridine-d₅)δ:: 71.0(d), 60.7(d), 54.1(d), 53.3(d), 43.2 (d), 41.8(t), 39.9(s), 39.5(t), 38.5(s), 38.4(s), 37.9(t), 37.3(s), 36.4(t), 35.9 (t), 35.6(t), 35.1(q), 33.2(t), 32.6(t), 32.3(q), 32.0(q), 30.9(t), 30.2(s), 28.3 (s), 20.3(q), 20.1(t), 18.8(q), 18.4(q), 18.3(t), 14.9(q), 10.7(q)

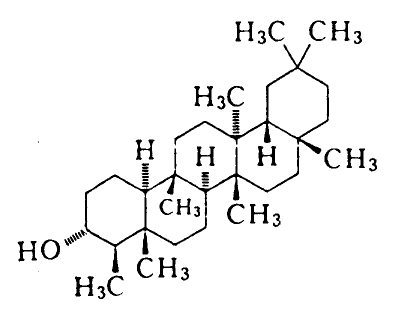

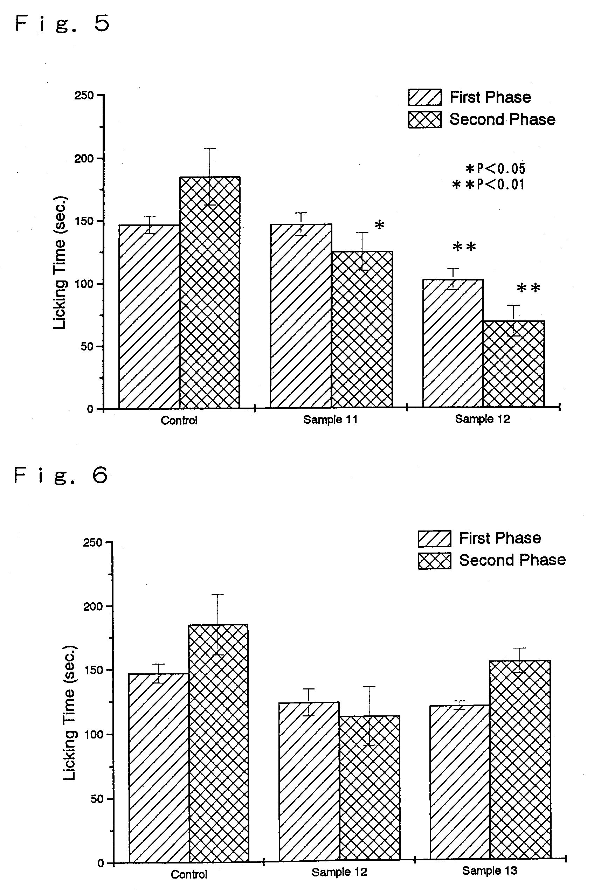

Formalin test (analgesic-anti-inflammatory effect test):
(a) 100ml of 2% formalin was injected as a stimulant into soles of rear paws (subcutaneous tissues of paw soles) of male IRC mice, which had been subjected to quarantine acclimation for one week after purchase at the age of 5 weeks, and total licking time of each mouse, i. e. duration of motions for a mouse to lick the rear paws to relieve the pain was measured by a stopwatch for each 5 minutes and continuously over 30 minutes. In the measurement, test drugs (Samples 11 and 12) were subcutaneously administered into mice at the time of 10 minutes before the stimulation, respectively, as 100µl of suspensions each containing such amounts of drugs in 0.5% polyoxyethylene-sorbitan monooleate (Tween 80, trademark of a product made by Atlas, Inc., USA), respectively, as to give a dose of 120mg/kg. Into the control mouse group, the same volume of aqueous 0.5% Tween 80 solution was subcutaneously administered.
Evaluation was carried out in two phases, as in the foregoing case (1), i. e. a first phase from 0 to 10 minutes and a second phase from 10 to 30 minutes, and total licking time in seconds, that is, duration of motions for a mouse to lick the rear paws, was measured and compared with the control.
Results of the formalin test for groups each of 5 mice are shown in the following Table 3 and Fig. 5.

**TABLE 3**

| | First phase of 0 - 10 mins. | Second phase of 10 - 30 mins. |
|---|---|---|
| Control | 146.95±7.10 secs. | 185.09±22.41 secs. |
| Sample 11 | 146.90±8.96 secs. | 124.96±15.05 secs. |
| Sample 12 | 102.54±8.56 secs. | 69.36±12.16 secs. |

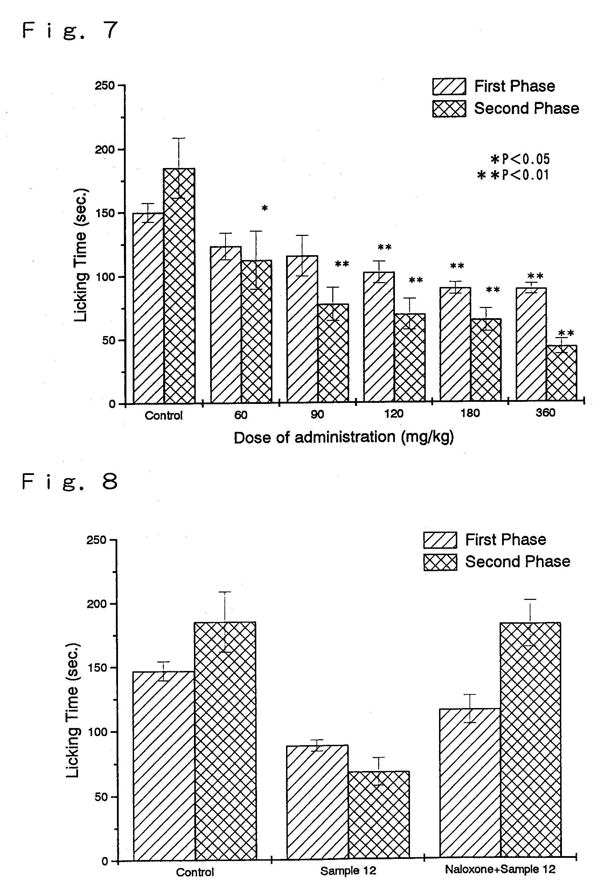

(b) Formalin test were carried out in the same manner as in (a) by subcutaneously administering Samples 12 and 13 as test drugs into mice, respectively, as 100µl of suspensions each containing such amounts of drugs in aqueous 0.5% Tween 80 solutions, respectively, as to give a dose of 60 mg/kg, and results as shown in the following Table 4 and Fig. 6 were obtained. Into the control mouse group, the same volume of aqueous 0.5% Tween 80 solution was subcutaneously administered.

**TABLE 4**

| | First phase of 0 - 10 mins. | Second phase of 10 - 30 mins. |
|---|---|---|
| Control | 146.95±7.48 secs. | 185.10±23.62 secs. |
| Sample 12 | 123.39±10.39 secs. | 112.32±22.71 secs. |
| Sample 13 | 119.88±3.29 secs. | 154.16±9.56 secs. |

(c) Formalin test were carried out in the same manner as in (a) except that the dose of administration of Sample 12 was changed to various degrees and results as shown in the following Table 5 and Fig. 7 were obtained. Into the control mouse group, the same volume of aqueous 0.5% Tween 80 solution was subcutaneously administered.

**TABLE 5**

| Dose (mg/kg) | First phase of 0 - 10 mins. | Second phase of 10 - 30 mins. |
|---|---|---|
| Control | 146.95±7.48 secs. | 185.10±23.62 secs. |
| 60 | 123.39+10.37 secs. | 112.32±22.71 secs. |
| 90 | 115.66±15.89 secs. | 77.49±13.10 secs. |
| 120 | 102.54±8.56 secs. | 69.36±12.16 secs. |
| 180 | 89.82±4.73 secs. | 64.90±9.10 secs. |
| 360 | 89.29±4.00 secs. | 43.78±5.99 secs. |

(d) It was found that Sample 12 effectively controlled both first and second phase reactions of formalin tests, and thus the formalin test was carried out in the same manner as in (a) after premedication of naroxone as a morphine antagonist. It is well known that action on the central nervous system typically by morphine can be antagonized by premedication of naroxone. It seems that the analgesic effect of the test drug is attenuated by the premedication, so that its participiation in the opioid receptor in the central nervous system and the peripheral nervous system is pronounced.
Naroxone was administered by subcutaneous injection of an aqueous solution containing such an amount of naroxone in physiological saline as to give a dose of 0.5 mg/kg at the time of 10 minutes before the administration of Sample 12. Sample 12 was administered by subcutaneous administration into mice as 100µl of a suspension containing such an amount of Sample 12 in an aqueous 0.5% Tween 80 solution as to give a dose of 180 mg/kg, thereby conducting the formalin test in the same manner as in (a) thereafter. Results shown in the following Table 6 and Fig. 8 were obtained. Into the control mouse group, the same volume of aqueous 0.5% Tween 80 solution was subcutaneously administered.

**TABLE 6**

| | First phase of 0 - 10 mins. | Second phase of 10 - 30 mins. |
|---|---|---|
| Control | 146.95±7.48 secs. | 185.10±23.62 secs. |
| Sample 12 | 88.27±4.37 secs. | 67.49±10.71 secs. |
| Naroxone (premedicated) + Sample 12 | 115.66±10.89 secs. | 182.40±18.10 secs. |

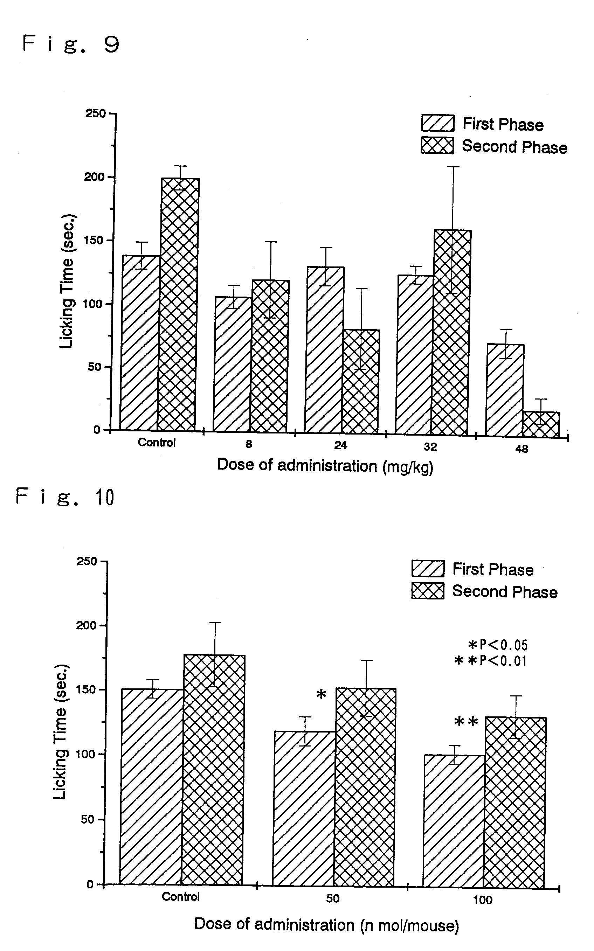

(e) 100µl each of suspensions each containing such amounts of Sample 12 in aqueous 0.5% Tween 80 solutions to give doses of 8, 24, 32 or 48 mg/kg, respectively, were prepared and subjected to the same formalin tests as in (a) except that the suspensions were administered into abdominal cavities of mice. Results shown in the following Table 7 and Fig. 9 were obtained. Into the control mouse group, the same volume of aqueous 0.5% Tween 80 solution was administered into the abdominal cavities.

**TABLE 7**

| Dose (mg/kg) | First phase of 0 - 10 mins. | Second phase of 10 - 30 mins. |
|---|---|---|
| Control | 138.64±10.70 secs. | 200.45±9.53 secs. |
| 8 | 107.50±9.15 secs. | 121.28±30.01 secs. |
| 24 | 132.13±15.13 secs. | 83.37±32.04 secs. |
| 32 | 126.76±7.01 secs. | 163.20±49.89 secs. |
| 48 | 74.19±11.66 secs. | 20.68±10.11 secs. |

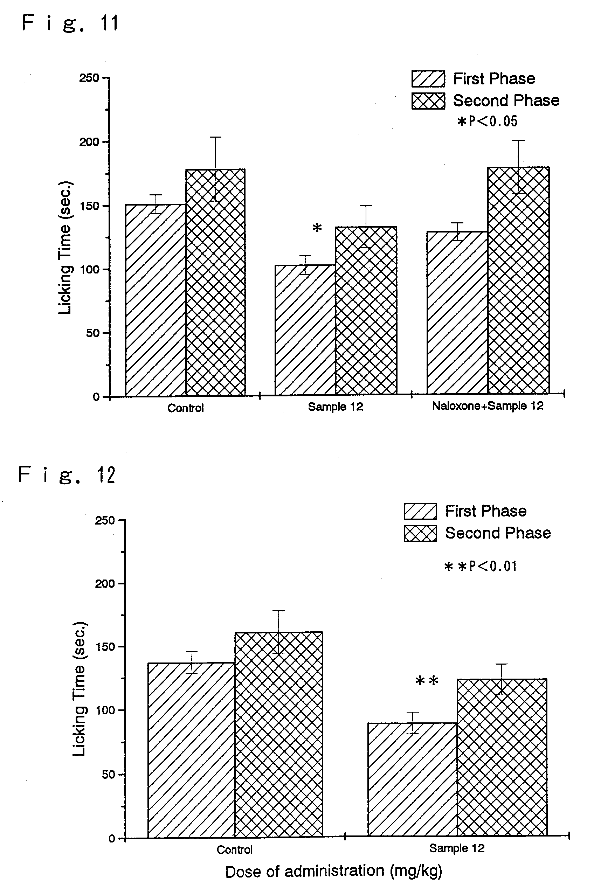

(f) 100µl each of suspensions each containing such amounts of Sample 12 in Ringer's solutions (containing 10% ethanol and 5% dimethylsulfoxide) as to give doses of 50 or 100 n mol/mouse, respectively, were prepared and subjected to formalin tests in the same manner as in (a) except that the suspensions were spinally administered at the time of 5 minutes before the formalin injection. Results shown in the following Table 8 and Fig. 10 were obtained. Into the control mouse group, the same volume of the Ringer's solution was spinally administered.

**TABLE 8**

| Dose (n mol) | First phase of 0 - 10 mins. | Second phase of 10 - 30 mins. |
|---|---|---|
| Control | 151.22±7.20 secs. | 178.21±25.00 secs. |
| 50 | 119.44±11.27 secs. | 153.16±21.43 secs. |
| 100 | 102.46±7.29 secs. | 132.09±16.54 secs. |

(g) It was found that the spinally administered Sample 12 effectively controlled both first and second phase reactions of formalin tests, and thus the formalin test was carried out in the same manner as in (f) after premedication of naroxone as a morphine antagonist. Naroxone was administered by subcutaneous administration of an aqueous solution containing such an amount of naroxone in physiological saline as to give a dose of 0.5 mg/kg at the time of 10 minutes before the administration of Sample 12. Sample 12 was administered at a dose of 100 n mol/mouse. Results shown in the following Table 9 and Fig. 11 were obtained. Into the control mouse group, the same volume of the Ringer's solution was spinally administered.

**TABLE 9**

| | First phase of 0 - 10 mins. | Second phase of 10 - 30 mins. |
|---|---|---|
| Control | 151.22±7.20 secs. | 178.21±25.00 secs. |
| Sample 12 | 102.46±7.29 secs. | 132.09±16.54 secs. |
| Naroxone (premedicated) + Sample 12 | 127.90±7.08 secs. | 178.47±20.83 secs. |

(h) 100µl of a suspension containing such an amount of Sample 12 in aqueous 0.5% Tween 80 solution as to give a dose of 360 mg/kg was prepared and subjected to the formalin test in the same manner as in (a) except that the suspensions was orally administered into mice at the time of 40 minutes before the formalin injection. Results shown in the following Table 10 and Fig. 12 were obtained. Into the control mouse group, the same volume of the aqueous 0.5% Tween 80 solution was orally administered.

**TABLE 10**

| | First phase of 0 - 10 mins. | Second phase of 10 - 30 mins. |
|---|---|---|
| Control | 137.34±8.66 secs. | 160.95±16.81 secs. |
| Sample 12 | 88.84±8.45 secs. | 122.94±11.79 secs. |

## Claims

1. An analgesic-anti-inflammatory drug which comprises an aqueous layer component obtained by intersolvent distribution of alcohol extract of Maytenus ilicifolia between chloroform and water as an effective component.

2. An analgesic-anti-inflammatory drug according to Claim 1, wherein the aqueous layer component is a 100% methanol eluate fraction from polystyrene-based resin column chromatography.

3. An analgesic-anti-inflammatory drug according to Claim 1, wherein the aqueous layer component is a 50% acetone eluate fraction from polystyrene-based resin column chromatography.

4. An analgesic-anti-inflammatory drug which comprises quercetin-3-0-glucoside as an effective component.

5. An analgesic-anti-inflammatory drug which comprises friedelin, friedelan-3-α-ol or friedelan-3-β-ol as an effective component.
